# EUROPÄISCHE PATENTANMELDUNG

(11) **EP 1 961 448 A2**
(43) Veröffentlichungstag der Anmeldung: **27.08.2008**
(21) Anmeldenummer: 08100131.5
(22) Anmeldetag: 07.01.2008
(51) Int. Cl.: A61Q 3/02, A61K 8/58

(54) **Glänzender und kratzfester Nagellack durch Zusatz von Silanen**

(30) Priorität: 26.02.2007 DE 102007009589
(71) Anmelder: Evonik Degussa GmbH, 45128 Essen (DE)
(72) Erfinder: Bergandt, Heike, 45772, Marl (DE); Weinelt, Frank, 48727, Billerbeck (DE)

(57) **Zusammenfassung**

Gegenstand der vorliegenden Erfindung ist ein Nagellack-Additiv, das
a) mindestens ein Silan der allgemeinen Formel 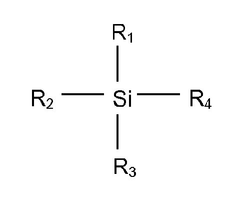 wobei die Reste R₁, R₂, R₃, R₄ gleich oder ungleich mit 1 bis 40 C-Atomen, und nicht-vemetzend sind, und
b) zumindest ein Lösemittel

aufweist, sowie ein Verfahren zur Herstellung des erfindungsgemäßen Nagcllack-Additivs und dessen Verwendung in einem Nagellack.

## Beschreibung

Die vorliegende Erfindung betrifft die Bereitstellung eines Additivs zur Verbesserung der Eigenschaften von Nagellacken, ein Verfahren zur Herstellung eines solchen Additivs, sowie dessen Verwendung in Nagellacken.

Ein moderner Nagellack dient der Erhaltung und Vermittlung einer ansprechenden Form und Färbung von Finger- und Fußnägeln. Zusätzlich wird der Nagel vor Umwelteinflüssen geschützt und erhält eine hohe Härte. Besondere Anstrengungen werden unternommen, um applizierte Nagellacke lange haltbar, kratzfest, splitterresistent, in attraktiver Färbung und ansprechendem Glanz zu gestalten.

Aktuelle Nagellacke bestehen aus einer Vielzahl von Inhaltstoffen. Als wichtigste Vertreter gehören dazu die Filmbildner, Haftvermittler, Weichmacher, Lösungsmittel und Pigmente. Zur Modifizierung der Rheologie und Thixotropie werden pyrogene Kieselsäuren eingesetzt. Trotz dieser breiten Möglichkeit, sehr gute Lacke zu formulieren, ist die Haltbarkeit, Härte und Kratzfestigkeit noch nicht zufrieden stellend.

Als Primärfilmbildner wird auch heute noch hauptsächlich Nitrocellulose eingesetzt, die im Wesentlichen für eine gute Härte und Zähigkeit der Lackoberfläche sorgt. Allerdings müssen, wie in DE 32 43 291 A1 offenbart wird, in aller Regel weitere Harze zugesetzt werden, um das Brechen und Absplittern zu reduzieren.

US 4873077, GB 1177420 und DE 69111621 offenbaren verschiedene Zusätze zur Verbesserung der Verschleißbeständigkeit, gegen das Splittern des Nagels, zur Vermeidung von Rissbildung, Abblättern und Bruch in Form von Synthese-, Natur- und mineralischen Fasern.

In der Schrift DE 4334938 A1 werden alkylverzweigte und polyglycoletherhaltige Fettsäureester für eine schnelle Trocknung mit harten und kratzfesteren Filmen empfohlen.

Zur Verbesserung des äußeren Erscheinungsbildes werden in der Anmeldung DE 198 22 722 A1 anorganisch-organische Hybridpolymere offenbart, die einer Nagellackformulierung zugegeben werden können. Dabei handelt es sich um organofunktionelle Silane, die am Siliciumatom drei kondensierbare und hydrolysierbare Funktionen tragen, sowie über einen vernetzbaren organischen Rest im Molekül verfügen. Als besonders bevorzugte Trockentemperaturen werden 40 bis 50°C genannt. Die Zeit zum Trocknen soll 2 bis 20 min betragen.

Durch Copolymere aus Acrylaten und Methacrylaten, offenbart in FR 7614430, sowie Copolymere mit polaren Funktionen, offenbart in DE 31 12 888 C2, sollen Brüchigkeit des getrockneten Nagellacks verringert und dessen Haftung auf dem Nagel verbessert werden.

Allen diesen Schriften ist gemeinsam, dass zur Verbesserung der Eigenschaften von Nagellacken diesen eine Vielzahl von Substanzen hinzugefügt wird, die dem fertigen kosmetischen Produkt in einem aufwendigen Verfahren beigemischt werden. Bei der schon seit längerer Zeit zunehmenden Sensibilität des Verbrauchers gegenüber Nebenwirkungen ist auch zu beobachten, dass der Kunde von der Anwendung von Nagellacken gänzlich absehen wird, wenn er wegen der gemäß Stand der Technik beigemischten Acrylat-Monomere mit Allergie auslösenden Wirkungen rechnen muss.

Aufgabe der vorliegenden Erfindung war es daher, ein Additiv bereit zu stellen, welches auf einfache Weise dem Nagellack beigemischt werden kann und welches die Nachteile des Standes der Technik überwindet.

Überraschend wurde gefunden, dass die kosmetischen Merkmale des Nagellacks durch einfaches Hinzufügen eines Nagellack-Additivs verbessert werden, das
a) mindestens ein Silan der allgemeinen Formel 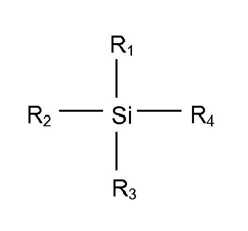
   wobei die Reste R₁, R₂, R₃, R₄ gleich oder ungleich mit 1 bis 40 C-Atomen, und nichtvemetzend sind,
   aufweist.

Gegenstand der vorliegenden Erfindung ist also ein Nagellack-Additiv, welches dadurch gekennzeichnet ist, dass das Nagellack-Additiv
a) mindestens ein Silan der allgemeinen Formel 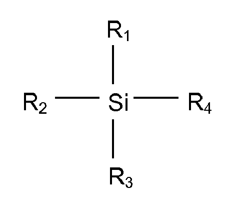 wobei die Reste R₁, R₂, R₃, R₄ gleich oder ungleich mit 1 bis 40 C-Atomen, und nichtvemetzend sind,
aufweist.

Gegenstand der vorliegenden Erfindung ist ebenfalls ein Verfahren zur Herstellung eines Nagellack-Additivs, welches dadurch gekennzeichnet ist, dass eine Zusammensetzung hergestellt wird, die die Komponente a) enthält oder aus diesen Komponenten besteht.

Gegenstand der vorliegenden Erfindung ist ebenso ein Nagellack-Additiv, das nach dem erfindungsgemäßen Verfahren erhalten wird.

Die vorliegende Erfindung hat den Vorteil, dass der mit dem erfindungsgemäßen Nagellack-Additiv versetzte Nagellack zu einem hochglänzenden und brillianten Überzug auf dem Nagel trocknet. Die Trocknungszeit des mit dem erfindungsgemäßen Nagellack-Additiv versetzten Nagellackes ist mit der Trocknungszeit der Nagellacke gemäß Stand der Technik vergleichbar. Das erfindungsgemäße Nagellack-Additiv weist den Vorteil auf, dass bei der Aushärtung das Silan oder die Silane nach dem Auftragen auf die Nageloberfläche mit der in der Luft zur Verfügung stehenden Feuchtigkeit unter Hydrolyse und Kondensation reagiert oder reagieren. Für den Anwender ergibt sich dadurch der weitere Vorteil, dass der Nagellack mit dem erfindungsgemäßen Nagellack-Additiv auf die Nägel aufgetragen werden kann, ohne seinem Anwender eine Umgewöhnung zum Beispiel in Gestalt längerer Wartezeiten oder besonderer Auftragungstechniken außer den gewohnten abzufordern.

Neben der schnellen Trocknung ist die Haftung auf dem Nagel sowie die Entfernbarkeit von der Nageloberfläche eine kritische Produkteigenschaft. Vergleichstests mit Nagellack gemäß Stand der Technik haben überraschend gezeigt, dass der mit dem erfindungsgemäßen Nagellack-Additiv versehene Lack ebenso gut oder besser auf der Oberfläche haftet und dennoch mit konventionellem Nagellackentferner ebenso einfach und schnell entfernbar ist, wie getrockneter Nagellack gemäß Stand der Technik.

Ein besonderer Vorteil des erfindungsgemäßen Nagellack-Additivs ist darin zu sehen, dass die mit diesem versetzten Nagellacke eine höhere Härte und verbesserte Kratzfestigkeit gegenüber Nagellack gemäß Stand der Technik aufweisen. Weiterhin ist neben der hohen Härte auch eine gewisse Flexibilität der lackierten Nageloberfläche wichtig, um die Bewegungen des Nagels mit zu machen und dabei möglichst wenig zum Absplittern und Abbrechen neigen. Mit erfindungsgemäßem Nagellack-Additiv versetzter Nagellack weist den Vorteil auf, dass der getrocknete Nagellack im Vergleich mit dem Stand der Technik weniger oder gar nicht absplittert oder abbricht.

Daher ist auch Gegenstand der vorliegenden Erfindung die Verwendung des erfindungsgemäßen Nagellack-Additivs in einem Nagellack.

Weiterhin ist Vorteil der vorliegenden Erfindung, dass der Anteil an filmbildenden Komponenten wie Nitrocellulose und/oder Polyacrylaten, die allergisch sensibilisierend wirken können, im Nagellack durch die Zugabe des Silans oder der Silane reduziert oder ganz auf solche filmbildenden Komponenten verzichtet werden kann. Wird bereits das Leistungsprofil selbst eines herkömmlichen Nagellacks durch die Zugabe des erfindungsgemäßen Nagellack-Additivs signifikant verbessert, wobei als ein Vorteil der vorliegenden Erfindung keinerlei unerwünschte Konkurrenzreaktionen mit den Stand der Technik gemäßen Komponenten auftreten, ist es ein ganz besonderer Vorteil der vorliegenden Erfindung, dass der Anteil dieser Komponenten, mit denen im Stand der Technik üblicherweise Härte, Glanz, Flexibilität und Kratzfestigkeit des konventionellen Nagellackes erreicht werden, reduziert werden kann. Härte, Glanz, Brillanz, Flexibilität und Kratzfestigkeit werden allein durch die Zugabe des erfindungsgemäßen Nagellack-Additivs in dem damit versehenen Nagellack erzielt und gegenüber den Merkmalen konventioneller Nagellacke sogar noch verbessert.

Somit ist ebenfalls Gegenstand der vorliegenden Erfindung ein Nagellack, der das erfindungsgemäße Nagellack-Additiv enthält oder ist, der nach der Applikation auf dem Nagel durch Reaktion mit der Luftfeuchte aushärtet. Weiterer Gegenstand der vorliegenden Erfindung ist daher auch die Verwendung des erfindungsgemäßen Nagellacks für Kosmetik und/oder Nagelpflege.

Der erfindungsgemäße Nagellack hat den Vorteil, durch die simultan verbesserte Flexibilität und Härte den Nagel besser vor Stößen und Umwelteinflüssen zu schützen. Ebenso vorteilhaft sind das Abbrechen und Absplittern der Nägel verringert.

Nachfolgend wird die vorliegende Erfindung beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Teilbereiche zum Offenbarungsgehalt der vorliegenden Erfindung gehören, ohne dass diese aus Gründen der besseren Übersichtlichkeit explizit genannt worden sind.

Gegenstand der vorliegenden Erfindung ist ein Nagellack-Additiv, dadurch gekennzeichnet, dass das Nagellack-Additiv
a) mindestens ein Silan der allgemeinen Formel 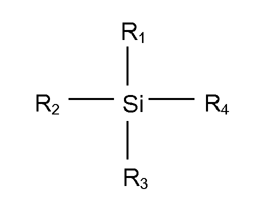
   wobei die Reste R₁, R₂, R₃, R₄ gleich oder ungleich mit 1 bis 40 C-Atomen, und nichtvemetzend sind,
aufweist.

Es kann vorteilhaft sein, wenn die Reste R₁, R₂, R₃, R₄ der Komponente a) des erfindungsgemäßen Nagellack-Additivs ausgewählt sind aus geradkettigen, verzweigten oder alicyklischen Alkyl-, Fluoralkyl-, Aryl-, Alkoxy-Gruppen, oder einer Kombination dieser Gruppen. Besonders bevorzugt ausgewählt sind Alkoxy-Gruppen. Vorzugsweise können die Alkoxy-Gruppen des erfindungsgemäßen Nagellack-Additivs ausgewählt sein aus methoxy-, ethoxy-, propoxy-, butoxy-, besonders bevorzugt ausgewählt sein aus ethoxy-, methoxy, propoxy-, ganz besonders bevorzugt ethoxy, methoxy-, oder einer Kombination dieser Gruppen.

Es kann weiterhin vorteilhaft sein, wenn das erfindungsgemäße Nagellack-Additiv eine weitere Komponente b) zumindest ein Lösemittel aufweist.

Vorzugsweise kann die Komponente b) des erfindungsgemäßen Nagellack-Additivs ausgewählt sein aus der Reihe der Alkohole der allgemeinen Formel CₙH₂ₙ₊₁OH, wobei n = 1 bis 4, besonders bevorzugt n=2 oder 3, aus der Reihe der mehrwertigen Alkohole, aus Ketonen, Acetaten, Glykolether, Toluol, Xylol, oder aus einem Gemisch dieser Lösemittel. Besonders bevorzugt kann diese Komponente ausgewählt sein aus Gylokole, Aceton, Methylethylketon, Ethylacetat, Butylactat, Ethylenglycolmonomethylether, oder einem Gemisch dieser Lösemittel.

Gegenstand der vorliegenden Erfindung ist ebenfalls ein Verfahren zur Herstellung eines Nagellack-Additivs, dadurch gekennzeichnet, dass eine Zusammensetzung hergestellt wird, die die Komponente a) enthält oder aus diesen Komponenten besteht.

Es kann vorteilhaft sein, wenn in dem erfindungsgemäßen Verfahren eine Zusammensetzung hergestellt wird, die die weitere Komponente b) in einer Menge von 0 bis 50 Gew.-%, wobei die Mengenangabe jeweils auf die Zusammensetzung bezogen ist, und mit der Maßgabe, dass die Summe der Gewichtsanteile 100 % ergibt, enthält.

Vorzugsweise kann in dem erfindungsgemäßen Verfahren eine Zusammensetzung hergestellt werden, die die Komponente a) in einer Menge von 0,01 bis 99,9 Gew.-%, bevorzugt von 1 bis 95 Gew.-%, weiters bevorzugt von 2 bis 90 Gew.-%, besonders bevorzugt von 5 bis 75 Gew.-%, weiters besonders bevorzugt von 10 bis 70 Gew.-%, weiterhin besonders bevorzugt von 15 bis 68 Gew.-%, ganz besonders bevorzugt von 25 bis 65 Gew.-%, und die Komponente b) in einer Menge von 0,01 bis 49,9 Gew.-%, bevorzugt von 1 bis 45 Gew.-%, weiters bevorzugt von 2 bis 40 Gew.-%, besonders bevorzugt von 5 bis 35 Gew.-%, weiters besonders bevorzugt von 8 bis 38 Gew.-%, ganz besonders bevorzugt von 10 bis 35 Gew.-%, wobei die Mengenangabe jeweils auf die Zusammensetzung bezogen ist, und mit der Maßgabe, dass die Summe der Gewichtsanteile 100 % ergibt, enthält oder aus diesen Komponenten besteht.

Es kann vorteilhaft sein, wenn in dem erfindungsgemäßen Verfahren die Komponenten a) und b) durch Rühren vermischt werden. Besonders bevorzugt können in dem erfindungsgemäßen Verfahren die Komponenten durch Rühren in einem Propellerrührer, Schrägblattrührer, Scheibenrührer, Impellerrührer, Kreuzbalkenrührer, Ankerrührer, Blattrührer, Gitterrührer, Wendelrührer, Zahnscheibenrührer, Turbinenrührer, Halbmondrührer, oder Fächerrührer miteinander vermischt werden. Es kann vorteilhaft sein, wenn in dem erfindungsgemäßen Verfahren Rührtechniken eingesetzt werden, die wenig oder gar kein Umgebungsgas in die Zusammensetzung einbringen und/oder bei denen wenig Wärmeenergie in die Zusammensetzung eingetragen wird. Ganz besonders bevorzugt können in dem erfindungsgemäßen Verfahren Propellerrührer, Schrägblattrührer, Scheibenrührer, Impellerrührer, Kreuzbalkenrührer, Ankerrührer, Blattrührer, Gitterrührer, Wendelrührer, oder Zahnscheibenrührer, weiterhin ganz besonders bevorzugt Propellerrührer, Scheibenrührer, oder Impellerrührer eingesetzt werden.

Vorzugsweise können in dem erfindungsgemäßen Verfahren die Komponenten b) und a) mit geringen Schergeschwindigkeiten während einer Zeitdauer von 1 bis 10 min, bevorzugt von 2 bis 8 min, besonders bevorzugt von 3 bis 7 min, ganz besonders bevorzugt von 4 bis 6 min, außerordentlich besonders bevorzugt von 4,8 bis 5,2 min miteinander vermischt werden.

Es kann weiterhin vorteilhaft sein, wenn in dem erfindungsgemäßen Verfahren das Lösungsmittel vorgelegt und anschließend die Komponente a) unter Rühren dazu gegeben wird. Vorzugsweise können in dem erfindungsgemäßen Verfahren die Komponente b) und a) bei einer Temperatur von 1 bis 30°C, besonders bevorzugt von 5 bis 28°C, weiterhin besonders bevorzugt von 10 bis 25°C, ganz besonders bevorzugt von 18 bis 21,5°C, außerordentlich bevorzugt bei Raumtemperatur durch Rühren vermischt werden.

Gegenstand der vorliegenden Erfindung ist ebenfalls die Verwendung des erfindungsgemäßen Nagellack-Additivs in einem Nagellack.

Es kann vorteilhaft sein, wenn das erfindungsgemäße Nagellack-Additiv in einer Konzentration von 1 und 13 Gew.-%, bevorzugt von 1,5 bis 12 Gew.-%, besonders bevorzugt von 2 bis 11 Gew.-%, ganz besonders bevorzugt von 2,5 bis 10 Gew.-% in dem Nagellack verwendet wird. Die Gewichtsangaben sind im Rahmen der vorliegenden Erfindung auf das Gewicht des für den Anwender fertigen, für die Auftragung auf den Nagel bereiten Nagellacks bezogen. In einer vorteilhaften Ausprägung der erfindungsgemäßen Verwendung kann das erfindungsgemäße Nagellack-Additiv durch Rühren oder Schütteln mit dem Nagellack, der ein konventioneller Nagellack oder ein Nagellack ohne filmbildende Komponenten sein kann, in diesen Nagellack eingearbeitet sein. Die erfindungsgemäße Verwendung hat den Vorteil, dass der auf dem Nagel aufgebrachte getrocknete Nagellack Glanz, Brillanz, Härte, Abriebfestigkeit und Flexibilität simultan aufweist. Wenn vorzugsweise als Komponente a) Dynasylan A, Dynasylan TEOS, Dynasilan MTES, alle diese Silane erhältlich bei Degussa GmbH, Paul-Baumann-Strasse 1,45764 Marl, Trimethylethoxysilan, Phenylethyltriethoxysilan, oder ein Gemisch dieser Silane verwendet wird, hat die erfindungsgemäße Verwendung außerdem den Vorteil, dass der erhaltene Nagellack den kosmetisch beabsichtigten Farbton, verglichen mit dem Farbton des konventionellen Nagellackes, beibehält, so dass keine Neuformulierung bezüglich des Farbdesigns erforderlich ist.

Das erfindungsgemäße Nagellack-Additiv wird nachfolgend anhand einiger Beispiele näher erläutert, ohne dass die Erfindung auf diese Ausführungsformen beschränkt sein soll.

### Beispiel 1: Herstellung eines Nagellack-Additivs

30 g Dynasylan TEOS und 30 g Butylacetat wurden für eine Zeitdauer von 5 min in einem Propellerrührer bei Zimmertemperatur miteinander vermischt.

Anschließend wurde 10 Gew.-% des so erhaltenen Nagellack-Additivs mit 90 Gew.-% handelsüblichem Nagellack gegeben. Dabei wurde das Nagellack-Additiv durch Rühren bei Raumtemperatur innig mit dem Nagellack vermischt, bis eine homogene Verteilung erreicht war.

Die Trocknung des Nagellacks ging mit der Vernetzung bzw. Kondensation des Silans nach der Applikation des Nagellackes auf der Nageloberfläche durch Reaktion mit der Luftfeuchtigkeit einher.

### Beispiel 2:

Wie Beispiel 1, jedoch wurde als Nagellack-Additiv 45 g Dynasylan TEOS und 55 g Dynasylan MTES eingesetzt.

### Beispiel 3:

Wie Beispiel 1, jedoch wurde als Nagellack-Additiv 20 g Trimethylethoxysilan, 50 g Dynasylan TEOS, 10g Dynasylan MTES, und 20 g Isopropanol eingesetzt.

### Beispiel 4:

Wie Beispiel 2, jedoch wurde 5 Gew.-% Nagellack-Additiv mit 95 Gew.-% handelsüblichem Nagellack vermischt.

### Beispiel 5:

Wie Beispiel 3, jedoch wurde 2,5 Gew.-% Nagellack-Additiv mit 97,5 Gew.-% handelsüblichem Nagellack vermischt.

### Beispiel 6:

Wie Beispiel 1, jedoch wurde das Nagellack-Additiv mit einem nicht-konventionellen Nagellack vermischt. Der nicht-konventionelle Nagellack hatte die Zusammensetzung gemäß Tabelle 1.

**Tabelle 1.**

| | |
|---|---|
| 16 g | Nitrocellulose, erhalten von Firma Wolff Cellulosics GmbH & Co. KG |
| 10 g | Arylsulfonamid |
| 6 g | Acetyltributylcitrat, Campher |
| 1,5 g | Stearalkonium Bentonit |
| 1,5 g | Pigmente |
| 65 g | Butylacetat |

### Beispiel 7:

Wie Beispiel 1, jedoch wurde das Nagellack-Additiv mit einem nicht-konventionellen Nagellack vermischt. Der nicht-konventionelle Nagellack hatte die Zusammensetzung gemäß Tabelle 2.

**Tabelle 2.**

| | |
|---|---|
| 16,5 g | Nitrocellulose (Wolff Cellulosics GmbH & Co. KG) |
| 10,3 g | Arylsulfonamid |
| 6,2 g | Acetyltributylcitrat, Campher |
| 67 g | Butylacetat |

### Beispiel 8:

Wie Beispiel 1, jedoch wurde das Nagellack-Additiv mit einem nicht-konventionellen Nagellack vermischt. Der nicht-konventionelle Nagellack hatte die Zusammensetzung gemäß Tabelle 3.

**Tabelle 3.**

| | |
|---|---|
| 10 g | Nitrocellulose (Wolff Cellulosics GmbH & Co. KG) |
| 6 g | Styrene/Acrylatcopolymer |
| 3 g | Silanmischung aus Beispiel 1 |
| 6 g | Acetyltributylcitrat, Campher |
| 1,5 g | Stearalkonium Bentonit |
| 1,5 g | Pigmente |
| 36 g | Butylacetat |
| 36 g | Ethylacetat |

### Beispiel 9:

Wie Beispiel 1, jedoch wurde das Nagellack-Additiv mit einem nicht-konventionellen Nagellack vermischt. Der nicht-konventionelle Nagellack hatte die Zusammensetzung gemäß Tabelle 4.

**Tabelle 4.**

| | |
|---|---|
| 16,5 g | Nitrocellulose (Wolff Cellulosics GmbH & Co. KG) |
| 6 g | Styrene/Acrylatcopolymer |
| 3,0 g | Silanmischung aus Beispiel 2 |
| 6,2 g | Acetyltributylcitrat, Campher |
| 40 g | Butylacetat |
| 28,3 g | Ethylacetat |

### Beispiel 10:

Wie Beispiel 1, jedoch wurde das Nagellack-Additiv mit einem nicht-konventionellen Nagellack vermischt. Der nicht-konventionelle Nagellack hatte die Zusammensetzung gemäß Tabelle 5.

**Tabelle 5.**

| | |
|---|---|
| 15,0 g | Nitrocellulose (Wolff Cellulosics GmbH & Co. KG) |
| 9,5 g | Arylsulfonamid |
| 3,0 g | Silan Mischung aus Beispiel 3 |
| 5,7 g | Acetyltributylcitrat, Campher |
| 1,4 g | Stearalkonium Bentonit |
| 1,4 g | Pigmente |
| 64,0 g | Butylacetat |

### Beispiel 11:

Wie Beispiel 1, jedoch wurde das Nagellack-Additiv mit einem nicht-konventionellen Nagellack vermischt. Der nicht-konventionelle Nagellack hatte die Zusammensetzung gemäß Tabelle 6.

**Tabelle 6.**

| | |
|---|---|
| 15,7 g | Nitrocellulose (Wolff Cellulosics GmbH & Co. KG) |
| 9,8 g | Arylsulfonamid |
| 2,5 g | Silan Mischung aus Beispiel 1 |
| 5,9 g | Acetyltributylcitrat, Campher |
| 66,1 g | Butylacetat |

## Patentansprüche

1. Nagellack-Additiv, das
a) Mindestens ein Silan der allgemeinen Formel 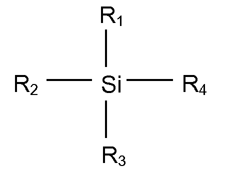 wobei die Reste R₁, R₂, R₃, R₄ gleich oder ungleich mit 1 bis 40 C-Atomen, und nicht-vernetzend sind,
aufweist.

2. Nagellack-Additiv nach Anspruch 1, das eine weitere Komponente
b) zumindest ein Lösemittel
aufweist.

3. Nagellack-Additiv nach zumindest einem der Ansprüche 1 oder 2,
**dadurch gekennzeichnet,**
**dass** die Reste ausgewählt sind aus geradkettigen, verzweigten oder alicyklischen Alkyl-, Fluoralkyl-, Aryl-, Alkoxy-Gruppe, oder einer Kombination dieser Gruppen.

4. Nagellack-Additiv nach zumindest einem der Ansprüche 1 bis 3,
**dadurch gekennzeichnet,**
**dass** die Alkoxy-Gruppe ausgewählt ist aus methoxy-, ethoxy-, propoxy-, butoxy-, oder einer Kombination dieser Gruppen.

5. Nagellack-Additiv nach zumindest einem der Ansprüche 1 bis 4,
**dadurch gekennzeichnet,**
**dass** die Komponente b) ausgewählt ist aus der Reihe der Alkohole der allgemeinen Formel CₙH₂ₙ₊₁OH, wobei n = 1 bis 4, oder einem Gemisch dieser Alkohole, oder aus der Reihe der mehrwertigen Alkohole, der Ketone, der Acetate, der Glykolether, Toluol, Xylol, oder einem Gemisch dieser Lösemittel.

6. Verfahren zur Herstellung eines Nagellack-Additivs,
**dadurch gekennzeichnet,**
**dass** eine Zusammensetzung hergestellt wird, die die Komponente a) enthält oder aus diesen Komponenten besteht.

7. Verfahren nach Anspruch 6,
**dadurch gekennzeichnet,**
**dass** eine Zusammensetzung hergestellt wird, die die weitere Komponente
b) in einer Menge von 0 bis 50 Gew.-%,
wobei die Mengenangabe jeweils auf die Zusammensetzung bezogen ist, und mit der Maßgabe, dass die Summe der Gewichtsanteile 100 % ergibt, enthält.

8. Verfahren nach zumindest einem der Ansprüche 6 oder 7,
**dadurch gekennzeichnet,**
**dass** die Komponenten durch Rühren vermischt werden.

9. Nagellack-Additiv, das nach dem Verfahren nach zumindest einem der Ansprüche 6 bis 8 erhalten wird.

10. Verwendung des Nagellack-Additivs nach zumindest einem der Ansprüche 1 - 5 oder 9 in einem Nagellack.

11. Nagellack, der das Nagellack-Additiv nach einem der Ansprüche 1 - 5 oder 9 enthält oder ist, der nach der Applikation auf dem Nagel durch Reaktion mit der Luftfeuchte aushärtet.

12. Verwendung eines Nagellacks nach Anspruch 11 für Kosmetik und/oder Nagelpflege.
